(19) Europäisches Patentamt / European Patent Office / Office européen des brevets

(11) **EP 4 025 121 B1**

(12) **EUROPEAN PATENT SPECIFICATION**

(45) Date of publication and mention of the grant of the patent:
**12.02.2025 Bulletin 2025/07**

(21) Application number: **20767990.3**

(22) Date of filing: **01.09.2020**

(51) International Patent Classification (IPC):
***A61B 5/021*** *(2006.01)*

(52) Cooperative Patent Classification (CPC):
**A61B 5/7221; A61B 5/021;** A61B 2560/0223

(86) International application number:
**PCT/EP2020/074256**

(87) International publication number:
**WO 2021/043729 (11.03.2021 Gazette 2021/10)**

(54) **DETECTION OF RELIABLE BLOOD PRESSURE MEASUREMENTS**

NACHWEIS VON ZUVERLÄSSIGEN BLUTDRUCKMESSUNGEN

DÉTECTION DE MESURES DE PRESSION ARTÉRIELLE FIABLES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.09.2019 US 201962894989 P**

(43) Date of publication of application:
**13.07.2022 Bulletin 2022/28**

(73) Proprietor: **Koninklijke Philips N.V.**
**5656 AG Eindhoven (NL)**

(72) Inventors:
• **SCHWAGER, Emma, Holdrich**
**5656 AE Eindhoven (NL)**
• **GHOSH, Erina**
**5656 AE Eindhoven (NL)**
• **LANIUS, Stephanie**
**5656 AE Eindhoven (NL)**
• **ESHELMAN, Larry, James**
**5656 AE Eindhoven (NL)**

(74) Representative: **Philips Intellectual Property & Standards**
**High Tech Campus 52**
**5656 AG Eindhoven (NL)**

(56) References cited:
WO-A1-2018/132352   WO-A1-2019/016802
US-A- 5 533 511   US-A1- 2010 081 944
US-A1- 2011 040 197   US-A1- 2018 078 155

**Description**

TECHNICAL FIELD

Embodiments described herein generally relate to systems and methods for determining the blood pressure of a patient and, more particularly but not exclusively, to systems and methods for determining accurate blood pressure measurements.

BACKGROUND

In a hospital environment, multiple types of blood pressure might be available for a single patient. A blood pressure measurement might come from an invasive or non-invasive measurement. For example, throughout most of a patient's stay in an intensive care unit, arterial blood pressure and non-invasive blood pressure are measured at the same time and the two measures generally agree.

However, during the course of a hospital stay, different types of blood pressure measurements may present different blood pressure values for a single patient. This may be addressed using averaging, which may be misleading, or by clinicians using clinical judgement to determine which blood pressure measurement to rely on. These subjective decisions may result in inaccurate measurements, leading to inaccurate diagnoses, procedural recommendations, and medication dosages.

A need exists, therefore, for methods and systems that overcome the above disadvantages of existing blood pressure determination techniques.

US 2018/078155 discloses a device capable of use in estimating blood pressure. The device includes one or more arterial sensors configured to obtain arterial measurements at two or more elevations. The device additionally includes one or more processors configured to determine one or more calibration parameters for a first blood pressure model based on the arterial measurements and a hydrostatic pressure difference between at least two of the elevations. The processors also are configured to determine a first blood pressure based on the first blood pressure model, the calibration parameters and the arterial measurements. The processors also are configured to determine a second blood pressure based in part on a second blood pressure model, one or more calibration parameters and the arterial measurements. The processors are further configured to provide a final blood pressure based on the first and second blood pressures.

US 2010/081944 discloses a method for recalibrating a non-invasive blood pressure monitor. Data corresponding to a patient is received and whether the blood pressure monitor needs to be recalibrated is determined based on the received data.

WO 2019/016802 discloses a wearable sensor array for measuring blood pressure. A pressure sensor array on a wrist band comprises a plurality of sensors configured to receive signals from all sensors simultaneously. One of the signals is selected as the blood pressure waveform for further processing or a composite signal made up of a weighted sum of all the signals is used to generate a blood pressure signal.

SUMMARY

This summary is provided to introduce a selection of concepts in a simplified form that are further described below in the Detailed Description section. This summary is not intended to identify or exclude key features or essential features of the claimed subject matter, nor is it intended to be used as an aid in determining the scope of the claimed subject matter.

The invention is defined by the claims. In one aspect, embodiments relate to a method for determining blood pressure of a patient. The method includes collecting, with a first device, a first plurality of blood pressure measurements of the patient; collecting, with a second device, a second plurality of blood pressure measurements of the patient; identifying, with a processor, a divergence between the first plurality and the second plurality; retrieving, from a memory, a clinical event; comparing, using the processor, the first plurality and the second plurality to the clinical event; and determining that the first plurality is more accurate than the second plurality based on comparison.

In some embodiments, identifying a divergence between the first plurality and the second plurality includes identifying, with a processor, a difference between the first plurality and the second plurality that persists for longer than a predetermined duration.

In some embodiments, identifying a divergence between the first plurality and the second plurality includes identifying, with a processor, a difference between the first blood pressure measurement and the second blood pressure measurement that exceeds a predetermined value.

In some embodiments, the clinical event is the administration of a blood pressure changing medication.

According to the invention, comparing blood pressure measurements to the at least one clinical event includes identifying a change in the measurements of the first plurality; and comparing a time of the identified change with a time of the clinical event. In some embodiments, determining that the first plurality is more accurate includes determining that the time of the identified change in the first plurality is roughly coincident with the time of the clinical event. According to the invention, comparing blood pressure measurements to the at least one clinical event includes comparing a directionality of the change in the measurements of the first plurality with a directionality expected from the clinical event. According to the invention, determining that the first plurality is more accurate includes determining that the directionality of the identified change in the first plurality is roughly concordant with the expected directionality of the clinical event.

In some embodiments, comparing the first plurality and the second plurality to the clinical event includes supplying the first plurality, the second plurality, and the clinical event to a trained machine learning model; and receiving, from the trained machine learning model, an indication that the second plurality is less accurate than the first plurality.

In some embodiments, identifying a difference between the first plurality and the second plurality includes at least one of using rule-based signal-quality indices, signal processing techniques, and machine learning algorithms.

According to another aspect, embodiments relate to a blood pressure assessment system. The system includes a first device configured to collect a first plurality of blood pressure measurements of the patient; a second device configured to collect a second plurality of blood pressure measurements of the patient; and a processor configured to identify a divergence between the first plurality and the second plurality; compare the first plurality and the second plurality to a clinical event; and determine that the first plurality is more accurate than the second plurality based on the comparison.

In some embodiments, the processor is further configured to identify a divergence between the first plurality and the second plurality by identifying a difference between the first plurality and the second plurality that persists for longer than a predetermined duration.

In some embodiments, the processor is further configured to identify a divergence between the first plurality and the second plurality by identifying a difference between the first blood pressure measurement and the second blood pressure measurement that exceeds a predetermined value.

In some embodiments, the clinical event is the administration of a blood pressure changing medication.

According to the invention, the processor is further configured to compare blood pressure measurements to the clinical event by identifying a change in the measurements of at least one of the first plurality and the second plurality; and comparing a time of the identified change with a time of the clinical event. In some embodiments, the processor is further configured to determine that the first plurality is more accurate by determining that the time of the identified change in the first plurality is roughly coincident with the time of the clinical event. According to the invention, the processor is further configured to compare blood pressure measurements to the clinical event by comparing a directionality of the change in the measurements of the first plurality with a directionality expected from the clinical event. According to the invention, the processor is further configured to determine that the first plurality is more accurate by determining that the directionality of the identified change in the first plurality is roughly concordant with the expected directionality of the clinical event.

In some embodiments, the processor is further configured to compare the first plurality and the second plurality to the clinical event by processing the first plurality, the second plurality, and the clinical event using a trained machine learning model; and receiving, from the trained machine learning model, an indication that the second plurality is less accurate than the first plurality.

In some embodiments, the processor is further configured to identify a difference between the first plurality and the second plurality using at least one of rule-based signal-quality indices, signal processing techniques, and machine learning algorithms.

<u>BRIEF DESCRIPTION OF DRAWINGS</u>

Non-limiting and non-exhaustive embodiments of the invention are described with reference to the following figures, wherein like reference numerals refer to like parts throughout the various views unless otherwise specified.

FIG. 1 illustrates a chart of the blood pressure measurements of a patient during the course of a hospital stay in accordance with one embodiment;

FIG. 2 illustrates a chart of diverging blood pressure measurements of a hospital patient in accordance with one embodiment;

FIG. 3 illustrates a method for measuring the blood pressure of a patient in accordance with one embodiment;

FIG. 4 illustrates a method for detecting reliable blood pressure measurements of patients in accordance with one embodiment;

FIG. 5 illustrates a schematic representation of a recurrent neural network architecture trained to forecast blood pressure using a given set of features in accordance with one embodiment;

FIG. 6 illustrates a chart of an arterial blood pressure measurement and a non-invasive blood pressure measurement diverging in accordance with one embodiment; and

FIG. 7 illustrates a cart of a blood pressure plateau between the arterial blood pressure and the non-invasive blood pressure in accordance with one embodiment.

<u>DETAILED DESCRIPTION</u>

Various embodiments are described more fully below with reference to the accompanying drawings, which form a part hereof, and which show specific exemplary embodiments. However, the concepts of the present disclosure may be implemented in many different forms and should not be construed as limited to the embodiments set forth herein; rather, these embodiments are provided as part of a thorough and complete disclosure, to fully convey the scope of the concepts, techniques and implementations of the present disclosure to those skilled in the art. Embodiments may be practiced as methods, systems or devices. Accordingly, embodiments may take the form of a hardware implementation, an entirely software implementation or an implementation combining software and hardware aspects. The following detailed description is, therefore, not to be taken in a limiting sense.

Reference in the specification to "one embodiment" or to "an embodiment" means that a particular feature, structure, or characteristic described in connection with the embodiments is included in at least one example implementation or technique in accordance with the present disclosure. The appearances of the phrase "in one embodiment" in various places in the specification are not necessarily all referring to the same embodiment. The appearances of the phrase "in some embodiments" in various places in the specification are not necessarily all referring to the same embodiments.

Some portions of the description that follow are presented in terms of symbolic representations of operations on non-transient signals stored within a computer memory. These descriptions and representations are used by those skilled in the data processing arts to most effectively convey the substance of their work to others skilled in the art. Such operations typically require physical manipulations of physical quantities. Usually, though not necessarily, these quantities take the form of electrical, magnetic or optical signals capable of being stored, transferred, combined, compared and otherwise manipulated. It is convenient at times, principally for reasons of common usage, to refer to these signals as bits, values, elements, symbols, characters, terms, numbers, or the like. Furthermore, it is also convenient at times, to refer to certain arrangements of steps requiring physical manipulations of physical quantities as modules or code devices, without loss of generality.

However, all of these and similar terms are to be associated with the appropriate physical quantities and are merely convenient labels applied to these quantities. Unless specifically stated otherwise as apparent from the following discussion, it is appreciated that throughout the description, discussions utilizing terms such as "processing" or "computing" or "calculating" or "determining" or "displaying" or the like, refer to the action and processes of a computer system, or similar electronic computing device, that manipulates and transforms data represented as physical (electronic) quantities within the computer system memories or registers or other such information storage, transmission or display devices. Portions of the present disclosure include processes and instructions that may be embodied in software, firmware or hardware, and when embodied in software, may be downloaded to reside on and be operated from different platforms used by a variety of operating systems.

The present disclosure also relates to an apparatus for performing the operations herein. This apparatus may be specially constructed for the required purposes, or it may comprise a general-purpose computer selectively activated or reconfigured by a computer program stored in the computer. Such a computer program may be stored in a computer readable storage medium, such as, but is not limited to, any type of disk including floppy disks, optical disks, CD-ROMs, magnetic-optical disks, read-only memories (ROMs), random access memories (RAMs), EPROMs, EEPROMs, magnetic or optical cards, application specific integrated circuits (ASICs), or any type of media suitable for storing electronic instructions, and each may be coupled to a computer system bus. Furthermore, the computers referred to in the specification may include a single processor or may be architectures employing multiple processor designs for increased computing capability.

The processes and displays presented herein are not inherently related to any particular computer or other apparatus. Various general-purpose systems may also be used with programs in accordance with the teachings herein, or it may prove convenient to construct more specialized apparatus to perform one or more method steps. The structure for a variety of these systems is discussed in the description below. In addition, any particular programming language that is sufficient for achieving the techniques and implementations of the present disclosure may be used. A variety of programming languages may be used to implement the present disclosure as discussed herein.

In addition, the language used in the specification has been principally selected for readability and instructional purposes and may not have been selected to delineate or circumscribe the disclosed subject matter. Accordingly, the present disclosure is intended to be illustrative, and not limiting, of the scope of the concepts discussed herein.

As mentioned previously, clinicians such as doctors, physicians, nurses, or other type of medical personnel often receive multiple types of blood pressure from a patient. In many retrospective databases of hospital data, multiple types of blood pressure are available for a single patient. In some embodiments, the blood pressure measurement could be an invasive blood pressure measurement or a non-invasive blood pressure measurement. In some embodiments, multiple types of invasive blood pressure measurements may be simultaneously taken for a patient. Sometimes, multiple types of blood pressure measurements may be taken simultaneously because the patient can have both a blood pressure cuff and one or more arterial lines available for measurements.

In some embodiments, multiple types of blood pressure measurements are used to measure the same quantity through different means. For example, arterial blood pressure may be measured both invasively and non-invasively. Any divergence between measurements might be an indication of a clinically relevant event and its automatic correct identification may enable the clinician to take faster and more effective action.

As explained earlier, when multiple blood pressure measurements are used to measure the same quantity through different means, analysts typically must choose a single measurement or combine multiple measurements through averaging to determine the true blood pressure of the patient. Eliminating incorrect blood pressure measurements is often a perquisite before applying other algorithms to detect disease, such as hemodynamic instability or acute kidney injury. In some embodiments, the method may be used in conjunction with disease-detecting algorithms to preprocess data from an electronic medical record before analysis. In some embodiments, an application of this method may reduce the number of false-positive alerts related to a patient's risk of disease.

In some embodiments, the system may use real-time monitoring to identify divergent blood pressure signals in real time. In some embodiments, the system may use predictive methods to alert that a divergence in blood pressure signals is likely for a patient. In some embodiments, the system may help a medical professional determine which blood pressure signal is most likely to be accurate. The system may then discard blood pressures determined to be inaccurate, such that a medical professional may obtain accurate blood pressure data regarding the patient.

In some embodiments, the system may use additional information from electronic medical records to select a blood pressure waveform from a plurality of blood pressure waveforms for diagnostic purposes. The electronic medical records may provide historical information and/or medical statistics about a plurality of patients. For example, in some embodiments, the electronic medical records may include drug use of the patient, previous blood pressure variations of the patient, and the duration of any blood pressure variations. In some embodiments, the use of drugs by a plurality of other patients and the effects of those drugs may also be used by a system to determine the accuracy of a type of blood pressure measurements.

In some embodiments, the system may ignore a blood pressure determined to be inaccurate and, in some embodiments, delete the incorrect blood pressure measurement from the medical records of the patient. In some embodiments, a blood pressure measurement determined to be incorrect may be corrected by the system after recording the inaccurate blood pressure. In some embodiments, all types of blood pressure measurements are recorded, but only the most accurate one may be used for future treatment of the patient. In some embodiments, the system may advise a clinician regarding the blood pressure of the patient and the reasons for selecting one type of blood pressure measurements. In some embodiments, the clinician may then convey the information from the system to the patient.

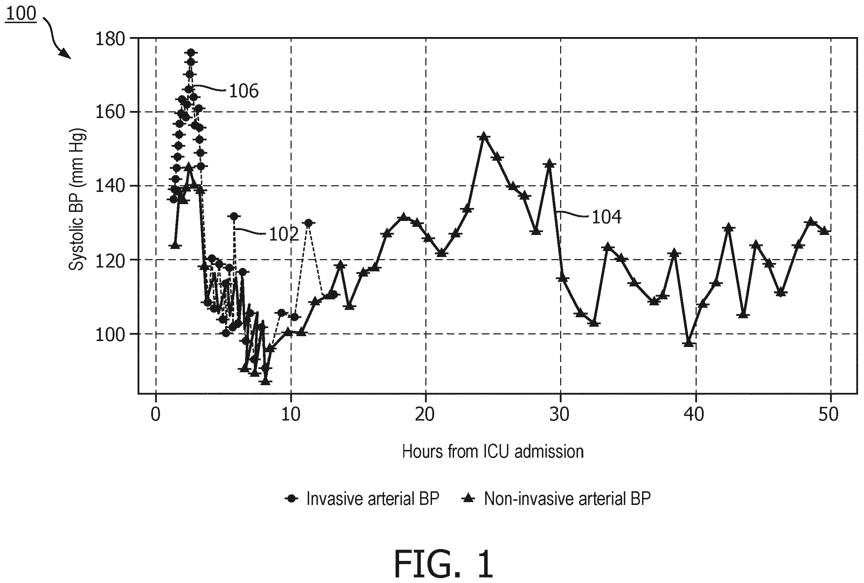

FIG. 1 illustrates a exemplary chart 100 of the blood pressure measurements of a patient during the course of a hospital stay in accordance with one embodiment. In some embodiments, systolic blood pressure measurements 102, 104 may be taken from a single patient throughout the course of their stay in a hospital. In some embodiments, the systolic blood pressure measurements are a non-invasive blood pressure measurement 104 and an arterial blood pressure measurement 102. In some embodiments, throughout the stay of the patient at the hospital, both the arterial blood pressure measurement 102 and the non-invasive blood pressure measurement 104 may correlate. A plateau can be observed in FIG. 1 at the beginning of the patient visit 106, wherein the arterial blood pressure measurement 102 is higher than the non-invasive blood pressure 104. As explained later, the plateau 106 can be expected and, in some embodiments, may be ignored during analysis of the patient blood pressure.

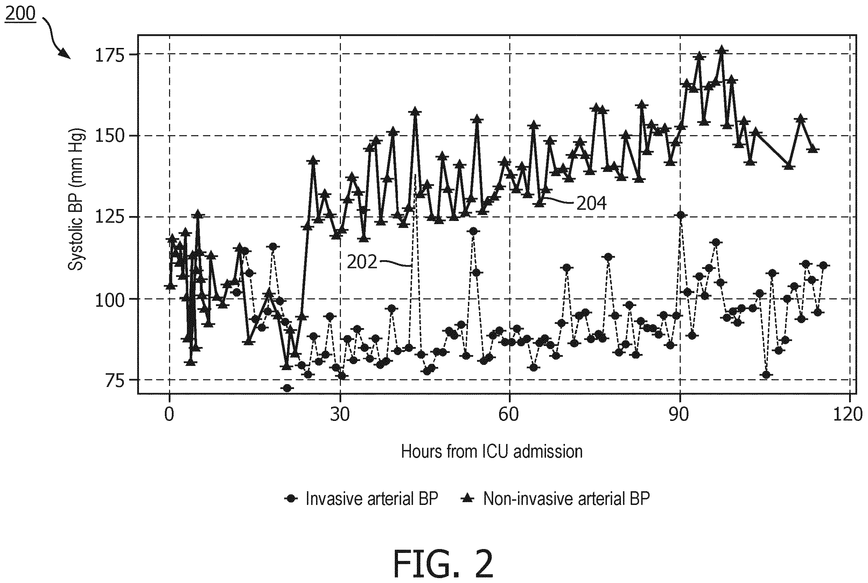

FIG. 2 illustrates an exemplary chart 200 of diverging blood pressure measurements 202, 204 in accordance with one embodiment. As illustrated, the arterial blood pressure measurement 202 is lower than the non-invasive blood pressure measurement 204 over the course of several days of monitoring. In some embodiments, the arterial blood pressure measurement 202 may be higher than the non-invasive blood pressure measurement 202. As may be inferred from the chart 200 and as discussed above, attempting to find the patient's "true" blood pressure by averaging the non-invasive blood pressure 204 and the arterial blood pressure 202 may result in misleading data. In some cases, at least one of the measured blood pressures is more accurate than the other. By weighting both of the measured blood pressures 202, 204 equally, an averaging calculation may lead to an inaccurate result.

As explained above, clinicians may use clinical judgment to determine the relative accuracy of the arterial blood pressure measurement 202 and the non-invasive blood pressure measurement 204. Simply averaging the two time series may lead to inaccurate results because, e.g., one of the time series measurements may be inaccurate.

In some embodiments, information from a database may be used to determine a reliable blood pressure measurement. This information may include the condition of the patient, the time of medication dosing, and medical procedures done with the patient. In some embodiments, it may not be clear to a physician or other medical professional which of the plurality of blood pressure measurements may be the most accurate for the patient.

In some embodiments, two blood pressure measurements must diverge for a certain length of time before a system uses only one blood pressure measurement to determine the blood pressure of a patient. In some embodiments, the length of time may be between one and six hours. In some embodiments, the length of time may be between five and thirty minutes. In some embodiments, the divergence monitoring may begin when two blood pressure measurements are simultaneously taken from a patient. In some embodiments, the divergence monitoring begins after a set amount of time to account for a potential plateau and expected divergence in blood pressure measurements at the beginning of a pressure assessment.

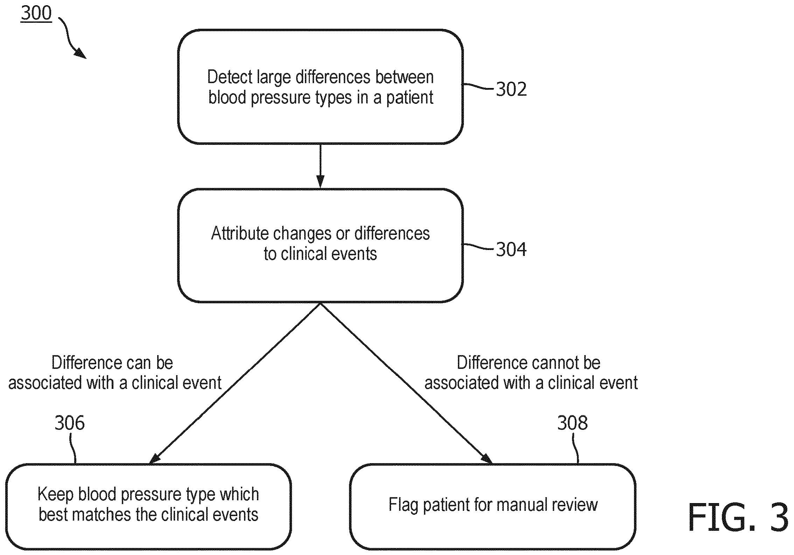

Once a divergence is detected in some embodiments, a system may use additional information to assess the reliability of various types of blood pressure measurements. FIG. 3 illustrates a method 300 for measuring the blood pressure of a patient using additional information in accordance with one embodiment.

In some embodiments, the system may detect large differences between blood pressure types in a patient 302. In some embodiments, the large differences may not occur for a significant length of time. If the system determines that the large differences between blood pressure types in a patient 302 are significant, the system may attempt to attribute changes or differences to clinical events 304.

In some embodiments, external information concerning the administration of medication, procedures done, and activity of the patient may be used to assess the reliability of a blood pressure measurement 304. For example, a system may collect information about whether a vasopressor or vasodilator was used by a patient around the time of the divergence. Additionally, the system may collect information regarding the length of time of divergence between two measurements. In some embodiments, the system may collect information regarding the magnitude of divergence between the two measurements.

The collected information may then be used to determine which collected blood pressure type matches the clinical events. For example, if a divergence occurred when the patient received a vasodilator, the blood pressure of the patient would be expected to decrease. If the divergence indicates one blood pressure measurement decreased while the other remained stable, the system may then proceed to keep and use the blood pressure measurement that decreased. In some embodiments, the blood pressure measurement that remained stable may be considered. If the system can associate the difference between blood pressure types with a known clinical event, the system may keep the blood pressure type which best matches the clinical event 306. In some embodiments, if the difference cannot be attributed to a clinical event, the patient may be flagged for manual review 308.

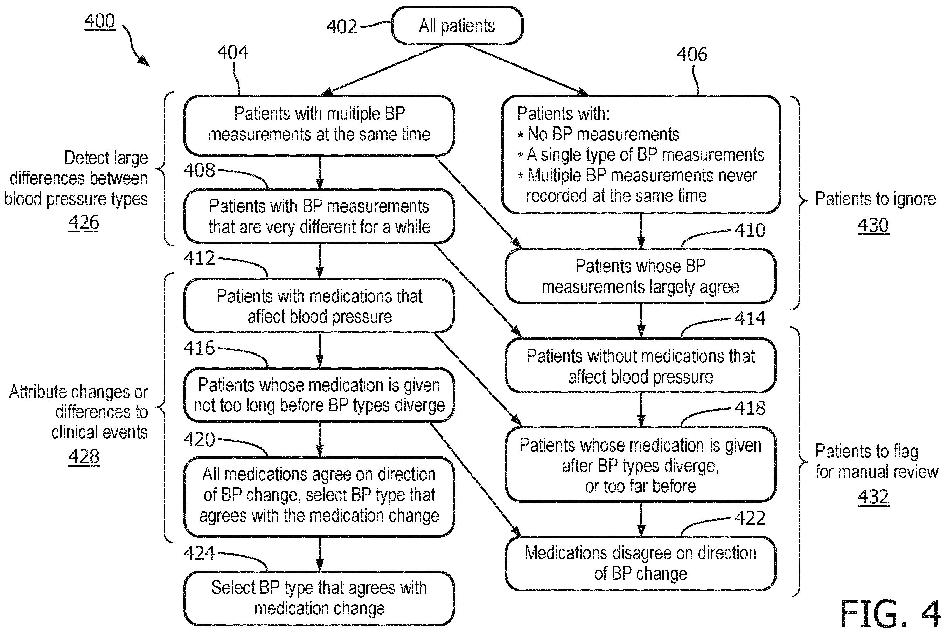

FIG. 4 illustrates a method 400 for detecting reliable blood pressure measurements of patients in accordance with one embodiment. In some embodiments, from the set of patients in a medical facility 402, the system may detect a subset of patients having multiple blood pressure measurements at the same time 404.

In some embodiments, the system may ignore the subset of patients with one or zero blood pressure measurements 406, as well as patients with blood pressure measurements that largely agree 410.

In some embodiments, some patients may have blood pressure measurements that diverge 408. In some embodiments, these may be referred to as patients with divergent blood pressure types. In some embodiments, patients with divergent blood pressure types may take medication that affects blood pressure 412. In some embodiments, if a patient with divergent blood pressure types was given medication that affects blood pressure within a timespan during which their blood pressure became divergent 416, and their medications affect blood pressure in the same way 420, the system may select the blood pressure type that agrees with the expected blood pressure change in some embodiments 424. In some embodiments, this may allow a system to attribute changes or differences in blood pressure of a patient to at least one clinical event 428.

In some embodiments, a patient with divergent blood pressure types may be flagged for manual review 432. In some embodiments, a patient having divergent blood pressure types may be flagged for manual review because the patient has not received any medications that affect blood pressure 414. In some embodiments, a patient having divergent blood pressure types may be flagged for manual review because the patient has received medications that disagree on the direction of the blood pressure change 422. For example, if a patient was given a vasodilator and then blood pressure increased, the patient may be flagged for manual review in some embodiments. Additionally, in some embodiments, a patient may be flagged for manual review if the patient received medication after the blood pressure types diverged or received medication too far before the divergence detection 418.

In some embodiments, if a blood pressure type $b$ at time $t$ for subject $i$ is $x_{it}^{b}$, a system may find all subjects with large differences between blood pressure types for a certain amount of time. In some embodiments, these patients are those for whom $\left| x_{it}^{b_1} - x_{it}^{b_2} \right| \geq C_i$ for $t \in [t_{i1}, t_{i2}]$ where $C_i$ is a (possibly subject-specific) cutoff, and $[t_{i1}, t_{i2}]$ is an interval of at least duration $T_i$. In some embodiments, duration $T_i$ may be a time interval during which two blood pressure type measurements diverged. In some embodiments, a patient may have three or more blood pressure type measurements diverging over $T_i$.

In some embodiments, among the patients with divergent types, the system may identify any medications affecting blood pressure given to the patient, the time of administration to the patient, and the expected direction of change in blood pressure for the patient.

For example, vasopressors are medications meant to increase blood pressure and vasodilators are medications meant to decrease blood pressure. Vasopressors increase blood pressure by constricting blood vessels and vasodilators decrease blood pressure by dilating blood vessels. If a patient were given a vasodilator and then the patient had divergent blood pressure types where the blood pressure in one blood measurement increased, in some embodiments, the patient would be flagged for manual review. Moreover, if a patient with divergent blood pressure types was given two or more medications and some medications may lead to an increased blood pressure, whereas others may lead to a decreased blood pressure, the patient would be flagged for manual review in some embodiments.

In some embodiments, a system includes a database of medications affecting blood pressure. In some embodiments, the database may be updated and maintained by clinical collaborators.

In mathematical terms, in some embodiments, the expected blood pressure direction of medication $m$ at time $t$ for subject $i$ is $y_{it}^m$ (+1 indicating an increase and -1 a decrease). For each medication $m$ and each subject $i$, the system may find $\{t_{i1}^m, \ldots, t_{iK}^m\}$, the set of times when the medication is administered. In some embodiments, $K$ may be the number of times of administration, and this set may be the empty set if a patient never receives the drug.

Next, in some embodiments, the system may align medication administration to a patient and blood pressure measurements of the patient. In some embodiments, if no medication was administered to the patient, further investigation might be warranted because other factors may be present to indicate the more reliable measurement.

In some embodiments, if all medications are administered after $t_{i1}$ in subject $i$ ($t_{i1}^m > t_{i1}$) for all medications $m$, then also no conclusions can be drawn and further investigation is warranted because the medication was administered after the divergence.

In some embodiments, for any medication administration before $t_{i1}$, the time of divergence, the system may collect all directional information within a certain time interval before $t_{i1}$: $\{y_{it}^m \mid t \in [t_{i0}, t_{i1}]\}$, where $[t_{i0}, t_{i1}]$ is a time interval of a pre-determined length (such as 3 hours). In some embodiments, the system may use the resulting directional information to determine which blood pressure is most reliable.

In some embodiments, if a single medication is given, then the system may use the higher blood pressure measurements if $y_{it}^m$ is +1, and the lower blood pressure measurements if $y_{it}^m$ is -1. In some embodiments, the system may use the higher blood pressure measurements if $y_{it}^m$ is +1, and the lower blood pressure measurements if $y_{it}^m$ is -1 if the patient has received multiple medications all having the same direction.

In some embodiments, if multiple medications with conflicting effects were given to a patient, additional information may need to be ascertained. Other factors may help, in some embodiments, to determine the accuracy of a blood pressure measurement, including but not limited to the strength of the effect of medication, how long its effect lasts, and other diagnoses of the patient. In some embodiments, the additional factors may be inputted into the system automatically and analyzed automatically. In some embodiments, additional factors may need to be manually assessed.

In some embodiments, machine-learning techniques may be used to detect reliable blood pressure measurements. In some embodiments, to identify patients that have large differences between blood pressure types, a system may use rule-based signal-quality indices, standard signal-processing techniques, and/or machine-learning algorithms. In some embodiments, to attribute the changes or differences to clinical events, a system may extract additional data and train a neural network to forecast blood pressure signals due to clinical events, such as dosing medication, the movement of a patient, or a scheduled procedure. In some embodiments, systems may extract concurrent clinical events such as lab values, medications, and information on interventions to identify potential factors which could influence the blood pressure measurement of a patient.

In some embodiments, data may be aligned with a blood pressure measurement time series to train a recurrent neural network (RNN) with long short-term memory (LSTM). In some embodiments, LSTM may include a standard forget gate wherein the system may determine the amount of past data to retain and rely upon when calculating predicted future blood pressure measurements. In some embodiments, the system may capture the temporal effect of medications and labs and use that captured effect to predict future blood pressure measurements and determine the accuracy of diverging blood pressure measurements.

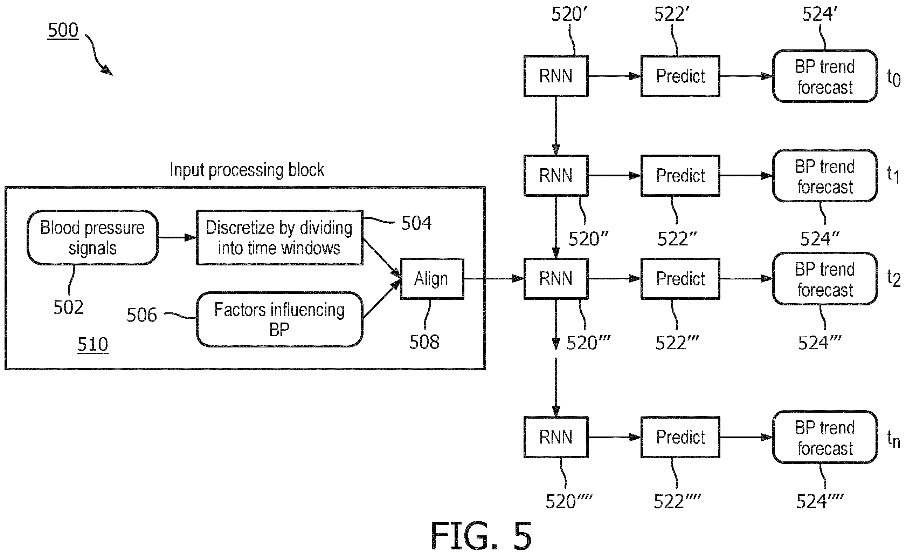

In some embodiments, a system may be trained to forecast a blood pressure signal for a given set of clinical events. FIG. 5 schematically illustrates a recurrent neural network architecture 500 trained to forecast blood pressure using a given set of features in accordance with one embodiment. In some embodiments, a forecasted signal 524 may be compared to the recorded signals to determine which is a better match to the expected trend. In some embodiments, the system may predict the divergence of blood pressure measurements of a patient.

In some embodiments, an input processing block 510 may receive a plurality of blood pressure signals 502. In some embodiments, the input processing block 510 may discretize the blood pressure signals by dividing them into windows 504. The discretized blood pressure signals may then, in some embodiments, be aligned 508 with factors influencing blood pressure 506. For example, in some embodiments, the time windows in which divergence of blood pressures occurs may be aligned 508 with the time a patient took vasodilator medications. In some embodiments, this information may be fed to a plurality of recurrent neural networks 520. In some embodiments, each recurrent neural network 520 may provide a predicted blood pressure measurement of a patient 522. In some embodiments, a system may generate a blood pressure trend forecast based on the prediction 524.

In some embodiments, recurrent neural networks 520 may send information to other recurrent neural networks 520 and the subsequent neural networks 520 may use additional information to predict the blood pressure of a patient.

In some embodiments, once a divergence in blood pressure measurements occurs, the system may detect the divergence and attempt to consolidate any disparities in the blood pressure measurements. In some embodiments, the system may determine the likelihood of accuracy between two or more blood pressure measurements. In some embodiments, the system may predict the likelihood of accuracy of blood pressure measurements. In some embodiments, the system may determine which of the plurality of blood pressure measurements may be first manually checked.

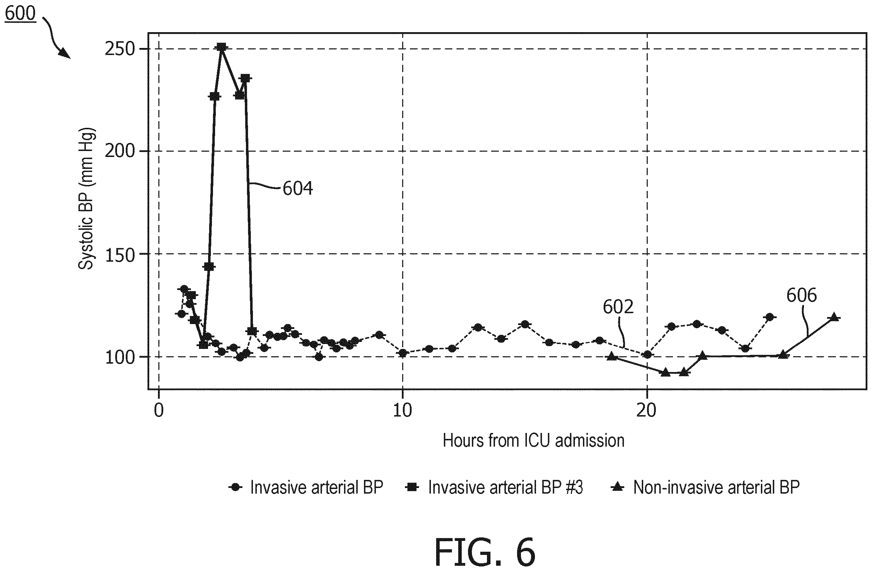

In some embodiments, the system may detect rapid termination of a collected blood pressure type. In some embodiments, a blood pressure may only be measured for a few hours before a physician or other medical professional ceases to measure it. As shown in FIG. 6, rapid termination of one blood pressure type 604 may indicate that a medical professional thought the collected blood pressure type was unreliable 600. In some embodiments, if a plurality of blood pressures 602-606 diverge and one is discontinued 604, the system may ignore divergent measurements of the continued blood pressure types 602, 606 for a period of time.

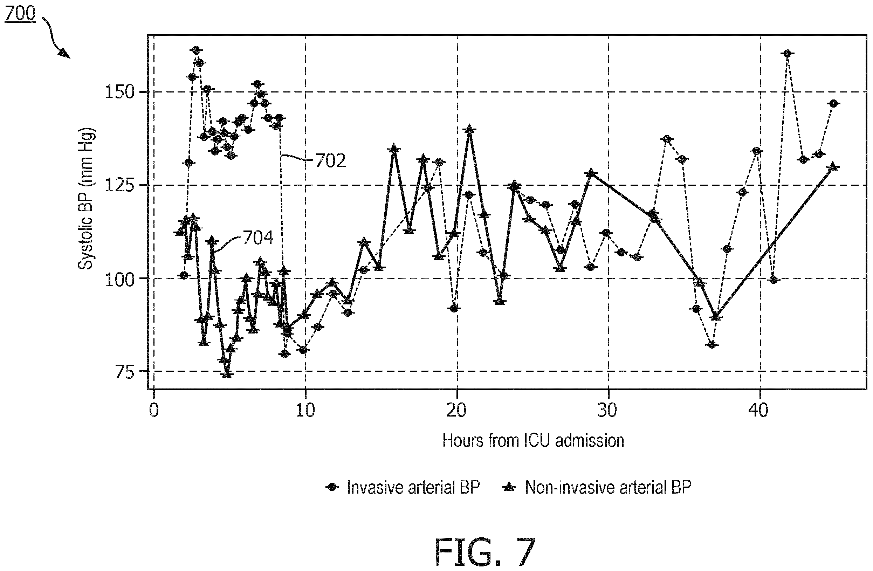

In some embodiments, divergence of blood pressure types 604 may be short and a plateau, especially at the beginning of the hospital visit. In some embodiments, a plateau 604 may indicate that the physician or other medical professional made an adjustment to the system. As is also shown in FIG. 7, the plateau 702 may occur for only a few hours for the patient before the blood pressure types converge 700. In some embodiments, the arterial blood pressure type 702 may initially register higher than the non-invasive blood pressure type 704. After a certain length of time, as can be shown by the chart 700, the plateau may resolve itself. In such a case, in some embodiments, the system may disregard one of the blood pressure measurements before the plateau resolved and may continue to use both blood pressure measurements after the plateau resolved. The methods, systems, and devices discussed above are examples. Various configurations may omit, substitute, or add various procedures or components as appropriate. For instance, in alternative configurations, the methods may be performed in an order different from that described, and that various steps may be added, omitted, or combined. Also, features described with respect to certain configurations may be combined in various other configurations. Different aspects and elements of the configurations may be combined in a similar manner. Also, technology evolves and, thus, many of the elements are examples and do not limit the scope of the disclosure or claims.

Embodiments of the present disclosure, for example, are described above with reference to block diagrams and/or operational illustrations of methods, systems, and computer program products according to embodiments of the present disclosure. The functions/acts noted in the blocks may occur out of the order as shown in any flowchart. For example, two blocks shown in succession may in fact be executed substantially concurrent or the blocks may sometimes be executed in the reverse order, depending upon the functionality/acts involved. Additionally, or alternatively, not all of the blocks shown in any flowchart need to be performed and/or executed. For example, if a given flowchart has five blocks containing functions/acts, it may be the case that only three of the five blocks are performed and/or executed. In this example, any of the three of the five blocks may be performed and/or executed.

A statement that a value exceeds (or is more than) a first threshold value is equivalent to a statement that the value meets or exceeds a second threshold value that is slightly greater than the first threshold value, e.g., the second threshold value being one value higher than the first threshold value in the resolution of a relevant system. A statement that a value is less than (or is within) a first threshold value is equivalent to a statement that the value is less than or equal to a second threshold value that is slightly lower than the first threshold value, e.g., the second threshold value being one value lower than the first threshold value in the resolution of the relevant system.

Specific details are given in the description to provide a thorough understanding of example configurations (including implementations). However, configurations may be practiced without these specific details. For example, well-known circuits, processes, algorithms, structures, and techniques have been shown without unnecessary detail in order to avoid obscuring the configurations. This description provides example configurations only, and does not limit the scope, applicability, or configurations of the claims. Rather, the preceding description of the configurations will provide those skilled in the art with an enabling description for implementing described techniques.

For example, the above elements may be components of a larger system, wherein other rules may take precedence over or otherwise modify the application of various implementations or techniques of the present disclosure. Also, a number of steps may be undertaken before, during, or after the above elements are considered.

Having been provided with the description and illustration of the present application, one skilled in the art may envision variations, modifications, and alternate embodiments falling within the general inventive concept discussed in this application that do not depart from the scope of the following claims.

**Claims**

1. A method for determining blood pressure of a patient, the method comprising:

   collecting, with a first device, a first plurality of blood pressure measurements (102, 202) of the patient;
   collecting, with a second device, a second plurality of blood pressure measurements (104, 204) of the patient;
   identifying (302), with a processor, a divergence between the first plurality (102, 202) and the second plurality (104, 204);
   retrieving, from a memory, a clinical event;
   analyzing (304), using the processor, the first plurality (102, 202) and the second plurality (104, 204) with reference to the clinical event, wherein analyzing blood pressure measurements with reference to the clinical event comprises:

      identifying a change in the measurements of the first plurality (102, 202);
      comparing a time of the identified change with a time of the clinical event; and
      comparing a directionality of the change in the measurements of the first plurality (102, 202) with a directionality expected from the clinical event, and

   determining (306) that the first plurality (102, 202) is more accurate than the second plurality (104, 204) based on the analyzing, wherein determining that the first plurality (102, 202) is more accurate comprises determining that the directionality of the identified change in the first plurality (102, 202) is roughly concordant with the expected directionality of the clinical event.

2. The method of claim 1 wherein identifying a divergence between the first plurality (102, 202) and the second plurality (104, 204) comprises identifying, with a processor, a difference between the first plurality (102, 202) and the second plurality (104, 204) that persists for longer than a predetermined duration.

3. The method of claim 1 wherein identifying a divergence between the first plurality (102, 202) and the second plurality (104, 204) comprises identifying, with a processor, a difference between the first blood pressure measurement (102, 202) and the second blood pressure measurement (104, 204) that exceeds a predetermined value.

4. The method of claim 1 wherein the clinical event is the administration of a blood pressure changing medication.

5. The method of claim 1 wherein determining that the first plurality (102, 202) is more accurate comprises determining that the time of the identified change in the first plurality (102, 202) is roughly coincident with the time of the clinical event.

6. The method of claim 1 wherein analyzing the first plurality (102, 202) and the second plurality (104, 204) with reference to the clinical event comprises:

   supplying the first plurality (102, 202), the second plurality (104, 204), and the clinical event to a trained machine learning model; and
   receiving, from the trained machine learning model, an indication that the second plurality (104, 204) is less accurate than the first plurality (102, 202).

7. The method of claim 1 wherein identifying a difference between the first plurality (102, 202) and the second plurality (104, 204) comprises at least one of using rule-based signal-quality indices, signal processing techniques, and machine learning algorithms.

8. A blood pressure assessment system, the system comprising:

   a first device configured to collect a first plurality of blood pressure measurements (102, 202) of the patient;
   a second device configured to collect a second plurality of blood pressure measurements (104, 204) of the patient; and
   a processor configured to:

      identify (302) a divergence between the first plurality (102, 202) and the second plurality (104, 204);
      analyze (304) the first plurality (102, 202) and the second plurality (104, 204) with reference to a clinical event, wherein analyzing blood pressure measurements with reference to the clinical event comprises:

identifying a change in the measurements of the first plurality (102, 202); comparing a time of the identified change with a time of the clinical event; comparing a directionality of the change in the measurements of the first plurality (102, 202) with a directionality expected from the clinical event, and

determine (306) that the first plurality (102, 202) is more accurate than the second plurality (104, 204) based on the analyzing, wherein determining that the first plurality (102, 202) is more accurate comprises determining that the directionality of the identified change in the first plurality (102, 202) is roughly concordant with the expected directionality of the clinical event.

9. The system of claim 8 wherein the processor is further configured to identify a divergence between the first plurality (102, 202) and the second plurality (104, 204) by identifying a difference between the first plurality (102, 202) and the second plurality (104, 204) that persists for longer than a predetermined duration.

10. The system of claim 8 wherein the processor is further configured to identify a divergence between the first plurality (102, 202) and the second plurality (104, 204) by identifying a difference between the first blood pressure measurement (102, 202) and the second blood pressure measurement (104, 204) that exceeds a predetermined value.

11. The system of claim 8 wherein the clinical event is the administration of a blood pressure changing medication.

12. The system of claim 8 wherein the processor is further configured to determine that the first plurality (102, 202) is more accurate by determining that the time of the identified change in the first plurality (102, 202) is roughly coincident with the time of the clinical event.

13. The system of claim 8 wherein the processor is further configured to analyze the first plurality (102, 202) and the second plurality (104, 204) with reference to the clinical event by:

processing the first plurality (102, 202), the second plurality (104, 204), and the clinical event using a trained machine learning model; and receiving, from the trained machine learning model, an indication that the second plurality (104, 204) is less accurate than the first plurality (102, 202).

14. The system of claim 8 wherein the processor is further configured to identify a difference between the first plurality (102, 202) and the second plurality (104, 204) using at least one of rule-based signal-quality indices, signal processing techniques, and machine learning algorithms.

**Patentansprüche**

1. Verfahren zum Bestimmen des Blutdrucks eines Patienten, wobei das Verfahren umfasst:

Sammeln, mit einer ersten Vorrichtung, einer ersten Vielzahl von Blutdruckmessungen (102, 202) des Patienten; Sammeln, mit einer zweiten Vorrichtung, einer zweiten Vielzahl von Blutdruckmessungen (104, 204) des Patienten; Identifizieren (302), mit einem Prozessor, einer Divergenz zwischen der ersten Vielzahl (102, 202) und der zweiten Vielzahl (104, 204); Abrufen, aus einem Speicher, eines klinischen Ereignisses; Analysieren (304), unter Verwendung des Prozessors, der ersten Vielzahl (102, 202) und der zweiten Vielzahl (104, 204) unter Bezugnahme auf das klinische Ereignis, wobei Analysieren von Blutdruckmessungen unter Bezugnahme auf das klinische Ereignis umfasst:

Identifizieren einer Veränderung in den Messungen der ersten Vielzahl (102, 202); Vergleichen eines Zeitpunkts der identifizierten Veränderung mit einem Zeitpunkt des klinischen Ereignisses; und Vergleichen einer Direktionalität der Veränderung in den Messungen der ersten Vielzahl (102, 202) mit einer Direktionalität, die von dem klinischen Ereignis erwartet wird, und

Bestimmen (306), dass die erste Vielzahl (102, 202) genauer ist als die zweite Vielzahl (104, 204), basierend auf der Analyse, wobei Bestimmen, dass die erste Vielzahl (102, 202) genauer ist, Bestimmen umfasst, dass die Direktionalität der identifizierten Veränderung in der ersten Vielzahl (102, 202) grob mit der erwarteten Direktionalität des klinischen Ereignisses übereinstimmt.

2. Verfahren nach Anspruch 1, wobei Identifizieren einer Divergenz zwischen der ersten Vielzahl (102, 202) und der zweiten Vielzahl (104, 204) Identifizieren, mit einem Prozessor, einer Differenz zwischen der ersten Vielzahl (102, 202) und der zweiten Vielzahl (104, 204), die länger als eine vorbestimmte Dauer anhält, umfasst.

3. Verfahren nach Anspruch 1, wobei Identifizieren einer Divergenz zwischen der ersten Vielzahl (102, 202) und der zweiten Vielzahl (104, 204) Identifizieren, mit einem Prozessor, einer Differenz zwischen der ersten Blutdruckmessung (102, 202) und der zweiten Blutdruckmessung (104, 204) umfasst, die einen vorbestimmten Wert überschreitet.

4. Verfahren nach Anspruch 1, wobei das klinische Ereignis die Verabreichung eines blutdruckverändernden Medikaments ist.

5. Verfahren nach Anspruch 1, wobei Bestimmen, dass die erste Vielzahl (102, 202) genauer ist, Bestimmen umfasst, dass der Zeitpunkt der identifizierten Veränderung in der ersten Vielzahl (102, 202) grob mit dem Zeitpunkt des klinischen Ereignisses zusammenfällt.

6. Verfahren nach Anspruch 1, wobei Analysieren der ersten Vielzahl (102, 202) und der zweiten Vielzahl (104, 204) unter Bezugnahme auf das klinische Ereignis umfasst:

Bereitstellen der ersten Vielzahl (102, 202), der zweiten Vielzahl (104, 204) und des klinischen Ereignisses für ein trainiertes Maschinenlernmodell; und
Empfangen, von dem trainierten Maschinenlernmodell, einer Angabe, dass die zweite Vielzahl (104, 204) weniger genau ist als die erste Vielzahl (102, 202).

7. Verfahren nach Anspruch 1, wobei Identifizieren einer Differenz zwischen der ersten Vielzahl (102, 202) und der zweiten Vielzahl (104, 204) mindestens eines von regelbasierten Signalqualitätsindizes, Signalverarbeitungstechniken und Algorithmen für maschinelles Lernen umfasst.

8. Blutdruckbewertungssystem, wobei das System umfasst:

eine erste Vorrichtung, die zum Sammeln einer ersten Vielzahl von Blutdruckmessungen (102, 202) des Patienten konfiguriert ist;
eine zweite Vorrichtung, die zum Sammeln einer zweiten Vielzahl von Blutdruckmessungen (104, 204) des Patienten konfiguriert ist; und
einen Prozessor, der konfiguriert ist zum:

Identifizieren (302) einer Divergenz zwischen der ersten Vielzahl (102, 202) und der zweiten Vielzahl (104, 204);
Analysieren (304) der ersten Vielzahl (102, 202) und der zweiten Vielzahl (104, 204) unter Bezugnahme auf ein klinisches Ereignis, wobei das Analysieren von Blutdruckmessungen unter Bezugnahme auf das klinische Ereignis umfasst:

Identifizieren einer Veränderung in den Messungen der ersten Vielzahl (102, 202);
Vergleichen eines Zeitpunkts der identifizierten Veränderung mit einem Zeitpunkt des klinischen Ereignisses;
Vergleichen einer Direktionalität der Veränderung in den Messungen der ersten Vielzahl (102, 202) mit einer Direktionalität, die von dem klinischen Ereignis erwartet wird, und

Bestimmen (306), dass die erste Vielzahl (102, 202) genauer ist als die zweite Vielzahl (104, 204), basierend auf der Analyse, wobei Bestimmen, dass die erste Vielzahl (102, 202) genauer ist, Bestimmen umfasst, dass die Direktionalität der identifizierten Veränderung in der ersten Vielzahl (102, 202) grob mit der erwarteten Direktionalität des klinischen Ereignisses übereinstimmt.

9. System nach Anspruch 8, wobei der Prozessor weiter konfiguriert ist, um eine Divergenz zwischen der ersten Vielzahl (102, 202) und der zweiten Vielzahl (104, 204) zu identifizieren, indem er eine Differenz zwischen der ersten Vielzahl (102, 202) und der zweiten Vielzahl (104, 204) identifiziert, die länger als eine vorbestimmte Dauer anhält.

10. System nach Anspruch 8, wobei der Prozessor weiter konfiguriert ist, um eine Divergenz zwischen der ersten Vielzahl (102, 202) und der zweiten Vielzahl (104, 204) zu identifizieren, indem er eine Differenz zwischen der ersten Blutdruckmessung (102, 202) und der zweiten Blutdruckmessung (104, 204) identifiziert, die einen vorbestimmten Wert überschreitet.

11. System nach Anspruch 8, wobei das klinische Ereignis die Verabreichung eines blutdruckverändernden Medikaments ist.

12. System nach Anspruch 8, wobei der Prozessor weiter konfiguriert ist, um zu bestimmen, dass die erste Vielzahl (102, 202) genauer ist, indem er bestimmt, dass der Zeitpunkt der identifizierten Veränderung in der ersten Vielzahl (102, 202) grob mit dem Zeitpunkt des klinischen Ereignisses zusammenfällt.

**13.** System nach Anspruch 8, wobei der Prozessor weiter konfiguriert ist, um die erste Vielzahl (102, 202) und die zweite Vielzahl (104, 204) unter Bezugnahme auf das klinische Ereignis zu analysieren, durch:

Verarbeiten der ersten Vielzahl (102, 202), der zweiten Vielzahl (104, 204) und des klinischen Ereignisses unter Verwendung eines trainierten Maschinenlernmodells; und
Empfangen, von dem trainierten Maschinenlernmodell, einer Angabe, dass die zweite Vielzahl (104, 204) weniger genau ist als die erste Vielzahl (102, 202).

**14.** System nach Anspruch 8, wobei der Prozessor weiter konfiguriert ist, um eine Differenz zwischen der ersten Vielzahl (102, 202) und der zweiten Vielzahl (104, 204) unter Verwendung mindestens eines von regelbasierten Signalqualitätsindizes, Signalverarbeitungstechniken und Algorithmen für maschinelles Lernen zu identifizieren.

**Revendications**

**1.** Procédé de détermination de la pression artérielle d'un patient, le procédé comprenant :

la collecte, avec un premier dispositif, d'une première pluralité de mesures de pression artérielle (102, 202) du patient ;
la collecte, avec un second dispositif, d'une seconde pluralité de mesures de pression artérielle (104, 204) du patient ;
l'identification (302), avec un processeur, d'une divergence entre la première pluralité (102, 202) et la seconde pluralité (104, 204) ;
la récupération, à partir d'une mémoire, d'un événement clinique ;
l'analyse (304), à l'aide du processeur, de la première pluralité (102, 202) et de la seconde pluralité (104, 204) en référence à l'événement clinique, dans lequel l'analyse de mesures de pression artérielle en référence à l'événement clinique comprend :

l'identification d'un changement dans les mesures de la première pluralité (102, 202) ;
la comparaison d'un temps du changement identifié avec un temps de l'événement clinique ; et
la comparaison d'une directionnalité du changement dans les mesures de la première pluralité (102, 202) avec une directionnalité attendue issue de l'événement clinique, et

la détermination (306) que la première pluralité (102, 202) est plus précise que la seconde pluralité (104, 204) sur la base de l'analyse, dans lequel la détermination que la première pluralité (102, 202) est plus précise comprend la détermination que la directionnalité du changement identifié dans la première pluralité (102, 202) est approximativement concordante avec la directionnalité attendue de l'événement clinique.

**2.** Procédé selon la revendication 1 dans lequel l'identification d'une divergence entre la première pluralité (102, 202) et la seconde pluralité (104, 204) comprend l'identification, avec un processeur, d'une différence entre la première pluralité (102, 202) et la seconde pluralité (104, 204) qui persiste pendant plus longtemps qu'une durée prédéterminée.

**3.** Procédé selon la revendication 1 dans lequel l'identification d'une divergence entre la première pluralité (102, 202) et la seconde pluralité (104, 204) comprend l'identification, avec un processeur, d'une différence entre la première mesure de pression artérielle (102, 202) et la seconde mesure de pression artérielle (104, 204) qui dépasse une valeur prédéterminée.

**4.** Procédé selon la revendication 1 dans lequel l'événement clinique est l'administration d'un médicament modifiant la pression artérielle.

**5.** Procédé selon la revendication 1 dans lequel la détermination que la première pluralité (102, 202) est plus précise comprend la détermination que le temps du changement identifié dans la première pluralité (102, 202) coïncide approximativement avec le temps de l'événement clinique.

**6.** Procédé selon la revendication 1 dans lequel l'analyse de la première pluralité (102, 202) et de la seconde pluralité (104, 204) en référence à l'événement clinique comprend :

la fourniture de la première pluralité (102, 202), de la seconde pluralité (104, 204) et de l'événement clinique à un modèle d'apprentissage automatique formé ; et
la réception, en provenance du modèle d'apprentissage automatique formé, d'une indication indiquant que la seconde pluralité (104, 204) est moins précise que la première pluralité (102, 202).

**7.** Procédé selon la revendication 1 dans lequel l'identification d'une différence entre la première pluralité (102, 202) et la seconde pluralité (104, 204) comprend au moins l'une parmi l'utilisation d'indices de qualité de signal basés sur des règles, de techniques de traitement de signal et d'algorithmes d'apprentissage automatique.

**8.** Système d'évaluation de la pression artérielle, le système comprenant :

un premier dispositif configuré pour collecter une première pluralité de mesures de pression artérielle (102, 202) du patient ;
un second dispositif configuré pour collecter une seconde pluralité de mesures de pression artérielle (104, 204) du patient ; et
un processeur configuré pour :

identifier (302) une divergence entre la première pluralité (102, 202) et la seconde pluralité (104, 204) ;
analyser (304) la première pluralité (102, 202) et la seconde pluralité (104, 204) en référence à un événement clinique, dans lequel l'analyse de mesures de pression artérielle en référence à l'événement clinique comprend :

l'identification d'un changement dans les mesures de la première pluralité (102, 202) ;
la comparaison d'un temps du changement identifié avec le moment de l'événement clinique ;
la comparaison d'une directionnalité du changement dans les mesures de la première pluralité (102, 202) avec une directionnalité attendue de l'événement clinique, et

la détermination (306) que la première pluralité (102, 202) est plus précise que la seconde pluralité (104, 204) sur la base de l'analyse, dans lequel la détermination que la première pluralité (102, 202) est plus précise comprend la détermination que la directionnalité du changement identifié dans la première pluralité (102, 202) est approximativement concordante avec la directionnalité attendue de l'événement clinique.

**9.** Système selon la revendication 8 dans lequel le processeur est en outre configuré pour identifier une divergence entre la première pluralité (102, 202) et la seconde pluralité (104, 204) en identifiant une différence entre la première pluralité (102, 202) et la seconde pluralité (104, 204) qui persiste pendant plus longtemps qu'une durée prédéterminée.

**10.** Système selon la revendication 8 dans lequel le processeur est en outre configuré pour identifier une divergence entre la première pluralité (102, 202) et la seconde pluralité (104, 204) en identifiant une différence entre la première mesure de pression artérielle (102, 202) et la seconde mesure de pression artérielle (104, 204) qui dépasse une valeur prédéterminée.

**11.** Système selon la revendication 8 dans lequel l'événement clinique est l'administration d'un médicament modifiant la pression artérielle.

**12.** Système selon la revendication 8 dans lequel le processeur est en outre configuré pour déterminer que la première pluralité (102, 202) est plus précise en déterminant que le temps du changement identifié dans la première pluralité (102, 202) coïncide approximativement avec le temps de l'événement clinique.

**13.** Système selon la revendication 8 dans lequel le processeur est en outre configuré pour analyser la première pluralité (102, 202) et la seconde pluralité (104, 204) en référence à l'événement clinique en :

traitant la première pluralité (102, 202), la seconde pluralité (104, 204) et l'événement clinique à l'aide d'un modèle d'apprentissage automatique formé ; et
recevant, en provenance du modèle d'apprentissage automatique formé, une indication indiquant que la seconde pluralité (104, 204) est moins précise que la première pluralité (102, 202).

**14.** Système selon la revendication 8 dans lequel le processeur est en outre configuré pour identifier une différence entre la première pluralité (102, 202) et la seconde pluralité (104, 204) à l'aide d'au moins un parmi des indices de qualité de signal basés sur des règles, des techniques de traitement de signal et des algorithmes d'apprentissage automatique.

FIG. 1

FIG. 2

EP 4 025 121 B1

300

Detect large differences between blood pressure types in a patient ——302

Attribute changes or differences to clinical events ——304

Difference can be associated with a clinical event

Difference cannot be associated with a clinical event

306

308

Keep blood pressure type which best matches the clinical events

Flag patient for manual review

FIG. 3

FIG. 4

FIG. 5

FIG. 6

FIG. 7

**EP 4 025 121 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2018078155 A
- US 2010081944 A
- WO 2019016802 A